# EUROPÄISCHE PATENTSCHRIFT

(11) **EP 3 318 238 B1**
(45) Veröffentlichungstag und Bekanntmachung des Hinweises auf die Patenterteilung: **13.02.2019**
(21) Anmeldenummer: 17200187.7
(22) Anmeldetag: 06.11.2017
(51) Int. Cl.: A61G 12/00, A61B 50/13

(54) **STATIONSWAGEN**
HOSPITAL CART
CHARIOT MÉDICAL

(30) Priorität: 08.11.2016 DE 202016106241 U
(43) Veröffentlichungstag der Anmeldung: 09.05.2018
(73) Patentinhaber: Optiplan Gesellschaft für optische Planungsgeräte mit beschränkter Haftung, 40489 Düsseldorf (DE)
(72) Erfinder: Wagner, Kai, 40489 Düsseldorf (DE); Lerner, Sabine, 40489 Düsseldorf (DE)
(74) Vertreter: Rausch Wanischeck-Bergmann Brinkmann

(56) Entgegenhaltungen:
- CN-U- 203 844 795
- DE-U1-202012 104 323
- GB-A- 2 106 455
- US-A1- 2002 096 845

## Beschreibung

Die Erfindung betrifft einen Stationswagen, insbesondere einen Visitenwagen für Krankenhäuser, Pflegeheime und sonstige Einrichtungen des Gesundheitswesens mit einem mittels Tragrollen fahrbar ausgebildeten Schrankunterteil, das der Aufnahme von Utensilien dient, und einem Schrankoberteil, das in Höhenrichtung relativ verfahrbar zum Schrankunterteil ausgebildet ist.

Stationswagen im Allgemeinen sowie in der speziellen Ausgestaltungsform als Visiten- und/oder Pflegewagen sind aus dem Stand der Technik an sich gut bekannt, so z. B. aus der DE 20 2013 104 564 U1 und der DE 20 2015 106 146 U1. Einen gattungsgemäßen Stationswagen offenbart die DE 20 2012 104 323 U1.

Die DE 196 42 523 A1 betrifft ebenfalls einen gattungsgemäßen Stationswagen mit einem Schrankkorpus, welcher nach oben hin eine abdeckende Ablage aufweist. Der Schrankkorpus besteht aus Seitenwänden, welche an den Ecken ein langgestrecktes Profil aufweisen, in welche Rollen eingesteckt sind.

Die DE 202 15 370 U1 zeigt einen Wagen für chirurgische Zwecke, dessen Rollen für die Verfahrbewegung an einer Bodenplatte angeordnet sind.

Obgleich sich Stationswagen der eingangs genannten Art im alltäglichen Praxiseinsatz bewährt haben, besteht das ständige Bestreben, die Handhabung derartiger Wagen konstruktionsbedingt weiter zu vereinfachen. Es ist deshalb die **Aufgabe** der Erfindung, einen gattungsgemäßen Stationswagen dahingehend weiterzuentwickeln, dass konstruktionsbedingt eine verwenderseitige Vereinfachung in der Handhabung erreicht ist.

Zur **Lösung** dieser Aufgabe wird mit der Erfindung ein Stationswagen der eingangs genannten Art vorgeschlagen, der sich dadurch auszeichnet, dass das Schrankunterteil eine Tragrahmenkonstruktion mit vertikal verlaufenden Stützstreben aufweist, wobei an jeder Stützstrebe jeweils ein Profilelement angeordnet ist, das einen Hohlraum bereitstellt, in den ein Zapfen einer Tragrolle eingesetzt ist, wobei eine die Tragrahmenkonstruktion tragrollenseitig abdeckende Platte vorgesehen ist, die zwischen den tragrollenseitigen Enden der Profilelemente und einem Rollenbock der jeweiligen an dem Profilelement angeordneten Tragrolle angeordnet ist.

Das Schrankunterteil verfügt über eine Tragrahmenkonstruktion, die quasi als selbsttragendes Chassis dient. Im bestimmungsgemäßen Verwendungsfall auftretenden Belastungen werden über diese Tragrahmenkonstruktion und die damit in Wirkverbindung stehenden Tragrollen in den Untergrund abgeleitet, auf dem der Stationswagen steht.

Die Tragrahmenkonstruktion verfügt über Stützstreben, die im bestimmungsgemäßen Anwendungsfall des erfindungsgemäßen Stationswagens vertikal verlaufen. Diese Stützstreben sind bevorzugterweise entlang der Eckkanten des Schrankunterteils verlaufend ausgebildet. Im endmontierten Zustand sind an den Stützstreben Seitenwandteile angebracht, die den vom Schrankunterteil bereitgestellten Aufnahmeraum begrenzen.

Je Stützstrebe ist ein Profilelement vorgesehen, das an der zugehörigen Stützstrebe angeordnet ist. Das Profilelement ist beispielsweise als Rundprofil ausgebildet, zumindest umfangsabschnittsweise. Das Profilelement stellt einen Hohlraum bereit, in den im endmontierten Zustand des Stationswagens ein Zapfen einer Tragrolle eingesetzt ist. Dabei weist die Tragrolle bevorzugterweise einen Rollenbock auf, an dem einerseits der Zapfen und andererseits Radscheiben verdrehbar angeordnet sind.

Im bestimmungsgemäßen Verwendungsfall steht der Stationswagen verfahrbar auf den am Schrankunterteil angeordneten Tragrollen. Dabei erfolgt im Belastungsfall die Kraftableitung über die Tragrahmenkonstruktion in die damit verbundenen Profilelemente und die daran angeordneten Tragrollen.

Der Stationswagen verfügt erfindungsgemäß über eine Platte, die die Tragrahmenkonstruktion tragrollenseitig abdeckt. Mittels dieser Platte ist die Tragrahmenkonstruktion nach unten hin verschlossen ausgebildet.

Die erfindungsgemäße Besonderheit besteht unter anderem darin, dass diese die Tragrahmenkonstruktion unterseitig abdeckende Platte zwischen den tragrollenseitigen Enden der Profilelemente einerseits und den Rollenböcken der an den Profilelementen angeordneten Tragrollen angeordnet ist.

Aufgrund der Zwischenordnung der Platte zwischen den Profilelementen einerseits und den Tragrollen andererseits ist sichergestellt, dass die Platte selbst kein die Konstruktion stützendes Element ist. Die Tragrahmenkonstruktion ist als selbsttragendes Chassis ausgebildet, das im Belastungsfall auftretende Kräfte über die daran angeordneten Profilelemente und die Tragrollen direkt ableitet. Die zwischen den Profilelementen und den Tragrollen angeordnete Platte trägt also weder zur Aussteifung der Gesamtkonstruktion bei, noch dient sie im bestimmungsgemäßen Verwendungsfall der Aufnahme und Ableitung von Belastungskräften.

Die erfindungsgemäße Konstruktion erweist sich herstellerseitig insofern von Vorteil, als dass eine vereinfachte Montage erreicht ist. Als nicht tragendes und im bestimmungsgemäßen Verwendungsfalle nicht kraftableitendes Bauteil bedarf es keiner besonderen Fixierung und/oder Montageschritte bei der Herstellung. Im einfachsten Fall verfügt die Platte über Durchgangsbohrungen, durch die hindurch im endmontierten Zustand die in die Profilelemente eingreifenden Zapfen der Tragrollen geführt sind. Die erfindungsgemäße Konstruktion gestattet es auch, die Platte im Bedarfsfall einfach austauschen zu können. Dies kann beispielsweise deshalb erforderlich sein, weil die Platte infolge einer bestimmungsgemäßen Benutzung im Laufe der Benutzungszeit beschädigt oder derart verschmutzt wird, dass ein optisch ansprechendes Reinigungsergebnis nicht mehr erreicht werden kann.

Verwenderseitig ist die erfindungsgemäße Konstruktion insbesondere insofern von Vorteil, als dass die Tragrollen von außen gut zugänglich sind, beispielsweise für ein Erreichen einer typischerweise fußbetätigbaren Feststellbremse. Darüber hinaus ist der zur Aufnahme von Utensilien schrankunterteilseitig bereitgestellte Volumenraum maximiert, da die Tragrollen an den Profilelementen angeordnet sind, die im endmontierten Zustand des Stationswagens quasi außerhalb des Schrankunterteils verlaufend ausgebildet sind. Von weiterem Vorteil ist in diesem Zusammenhang die robuste Gesamtkonstruktion, womit eine lange Lebensdauer gewährleistet ist. Insbesondere sorgt die Anordnung der Tragrollen an den Profilelementen dafür, dass eine dauerhaft sichere und haltbare Verbindung geschaffen ist. Eine solch sichere und haltbare Verbindung wird bei aus dem Stand der Technik bekannten Konstruktionen, gemäß welcher die untere Platte als tragendes Element dient, an dem die Tragrollen direkt angeordnet sind, nicht erreicht. Denn im Belastungsfall können die Tragrollen aus der Platte ausreißen, was bei der erfindungsgemäßen Konstruktion dank der Anordnung der Tragrollen an den Profilelementen nicht möglich ist.

Es ist gemäß einem weiteren Merkmal der Erfindung vorgesehen, dass die Platte die Tragrahmenkonstruktion unter Ausbildung von Überständen seitlich zumindest abschnittsweise überragt. Gemäß diesem Vorschlag der Erfindung ist also vorgesehen, dass die Platte im Grundriss größer als die Tragrahmenkonstruktion ist. Im endmontierten Zustand steht die Platte also über die Tragrahmenkonstruktion seitlich vor. Diese Ausgestaltung hat den Vorteil, dass der Stationswagen im Verfahrfall verwenderseitig mit dem Fuß betätigt werden kann. Die überstehende Platte dient dem Verwender als sicherer Aufsetzkant, was eine Betätigung mittels eines verwenderseitigen Fußes erleichtert.

Der Plattenüberstand ist gemäß einem weiteren Merkmal der Erfindung vorzugsweise im Bereich einer jeden Tragrolle bereitgestellt. Der Plattenüberstand wirkt im Bereich einer Tragrolle als Rollen-, mithin als Rammschutz. Zusätzliche Rammschutzeinrichtungen bedarf es gemäß dieser besonderen Ausgestaltung der Erfindung also nicht. Mit dieser Weiterbildung der Erfindung wird insofern auch ein synergetischer Effekt erreicht, da die Platte nicht nur dazu dient, die Tragrahmenkonstruktion unterseitig abzudecken, sondern sie dient gleichzeitig auch als Rammschutz.

Es ist gemäß einem weiteren Merkmal der Erfindung vorgesehen, dass die Tragrahmenkonstruktion plattenseitig einen zur Platte beabstandet angeordneten Innenboden trägt. Der Innenboden ist bevorzugterweise mit der Tragrahmenkonstruktion verbunden, vorzugsweise verschraubt, wodurch eine zusätzliche Aussteifung der Tragrahmenkonstruktion erreicht ist. Im bestimmungsgemäßen Verwendungsfall dient der Innenboden zur Bereitstellung der eigentlichen Aufnahmefläche zur Aufnahme von vom Schrankunterteil zu beherbergenden Utensilien. Damit stellt der Innenboden die verwenderseitig optisch wahrnehmbare Bodenfläche dar.

Der Innenboden ist zu der die Tragrahmenkonstruktion unterseitig abdeckenden Platte beabstandet angeordnet, so dass zwischen dem Innenboden und der Platte ein Hohlraum ausgebildet ist. Dieser Hohlraum dient gemäß einem weiteren Merkmal der Erfindung dazu, Kabel oder Kabelstränge aufzunehmen, wie sie typischerweise bei einer Ausstattung des Stationswagens mit einer Rechneranlage zu verlegen bzw. unterzubringen sind.

Weitere Merkmale und Vorteile der Erfindung ergeben sich aus der nachfolgenden Beschreibung anhand der Figuren. Dabei zeigen:
- Figur 1: in schematisch perspektivischer Darstellung einen erfindungsgemäßen Stationswagen und
- Figur 2: in schematisch perspektivischer Explosionsdarstellung ausschnittsweise Einzelteile des Schrankunterteils.

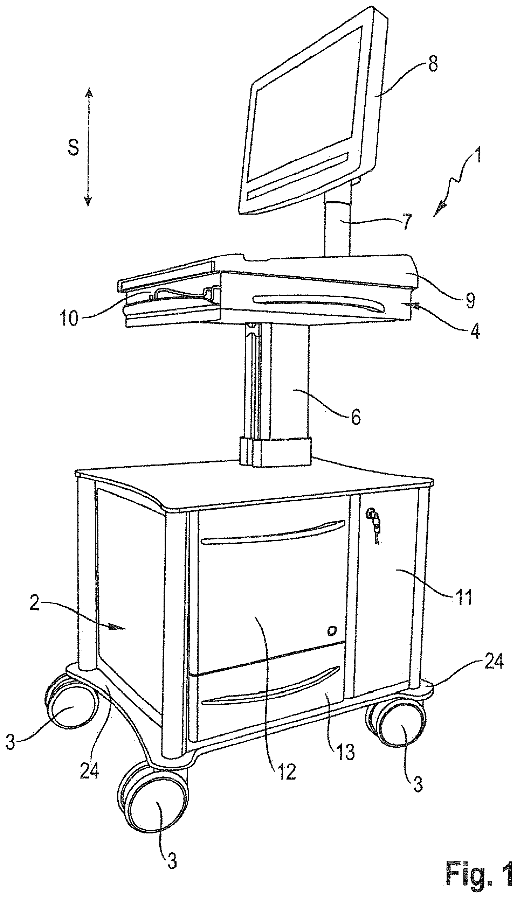

Figur 1 lässt den erfindungsgemäßen Stationswagen 1 in einer perspektivischen Ansicht erkennen. Dieser verfügt über ein Schrankunterteil 2 und ein Schrankoberteil 4. Das Schrankoberteil 4 ist von einer teleskopierbaren Säule 6 getragen, was es gestattet, das Schrankoberteil 4 in Höhenrichtung 5 relativ gegenüber dem Schrankunterteil 2 zu verfahren.

Der Stationswagen 1 ist verfahrbar ausgebildet, zu welchem Zweck Tragrollen 3 vorgesehen sind, die unterseitig am Schrankunterteil 2 angeordnet sind.

Im dargestellten Ausführungsbeispiel ist der Stationswagen 1 mit einer im Detail nicht näher dargestellten Computeranlage ausgerüstet.

Das Schrankunterteil 2 verfügt über ein Staufach 11, das mittels einer Tür verschließbar ist. Ferner verfügt das Schrankunterteil 2 über zwei Schubladen 12 und 13. Dabei stellt die Schublade 12 eine solche Größe bereit, die dafür geeignet ist, sogenannte Befundsammeltaschen aufnehmen zu können. Das Staufach 11 dient indes der Aufnahme einer Rechnereinheit.

Am Schrankoberteil 4 ist eine Säule 7 angeordnet, die endseitig einen Monitor 8 trägt. Dieser ist kommunikationstechnisch an die vom Staufach 11 beherbergte Rechnereinheit angeschlossen. An die Rechnereinheit sind ferner Peripheriegeräte, wie z. B. eine Maus oder eine Tastatur angeschlossen, die sich im Nicht-Verwendungsfall in einem vom Schrankoberteil 4 bereitgestellten Staufach 10 befinden, aus dem sie im Verwendungsfall herausverfahren werden können.

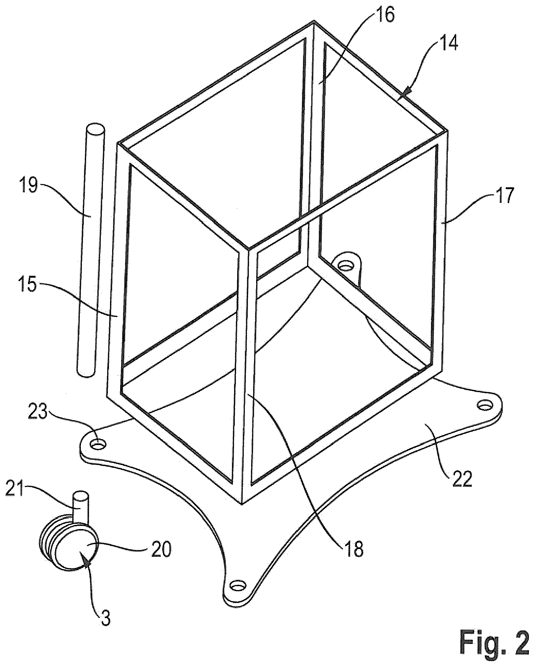

Das Schrankunterteil 2 verfügt über eine selbsttragende Tragrahmenkonstruktion 14, wie sie rein schematisch in Figur 2 dargestellt ist. Diese Tragrahmenkonstruktion 14 verfügt über vier Stützstreben 15, 16, 17 und 18, die jeweils eine Eckkante des Schrankunterteils 2 bilden. Im endmontierten Zustand nehmen die Stützstreben 15, 16, 17 und 18 daran angeordnete Seitenplatten auf, wie dies der endmontierte Zustand des Stationswagens 1 nach Figur 1 erkennen lässt.

Jeder der Stützstreben 15 bis 18 ist ein Profilelement 19 zugeordnet, wobei der besseren Übersicht wegen in Figur 2 nur das der Stützstrebe 15 zugeordnete Profilelement 19 zu erkennen ist. Dieses Profilelement 19 ist in Umfangsrichtung zumindest abschnittsweise kreisförmig ausgebildet. Es ist mit der jeweils zugehörigen Stützstrebe im endmontierten Zustand verschraubt, so dass eine feste und sichere Anordnung des Profilelements an der zugehörigen Stützstrebe gegeben ist.

Endseitig eines jeden Profilelements 19 ist eine Tragrolle 3 angeordnet. Eine Tragrolle 3 verfügt über einen Rollenbock 20, der einerseits verdrehbar Radscheiben aufnimmt sowie andererseits einen Zapfen 21 bereitstellt. Im endmontierten Zustand ist der Zapfen 21 einer jeden Tragrolle 3 in einen vom jeweiligen Profilelement 19 bereitgestellten Hohlraum eingesteckt. Zur Lagesicherung ist der Zapfen 21 mittels einer quer zum Zapfen 21 ausgerichteten Schraube mit dem Profilelement 19 verschraubt. Es ist so eine dauerhafte sichere und stabile und damit insgesamt robuste Gesamtkonstruktion geschaffen.

Die Tragrahmenkonstruktion 14 ist unterseitig mittels einer Platte 22 abgedeckt. Dabei ist die Platte 22 im endmontierten Zustand zwischen den tragrollenseitigen Enden der Profilelemente 19 einerseits und den Rollenböcken 20 der jeweiligen an den Profilelementen 19 angeordneten Tragrollen 3 angeordnet. Zu diesem Zweck verfügt die Platte 22 über Bohrungen 23, durch die hindurch die jeweils zugehörigen Zapfen 21 der Tragrollen 3 geführt sind.

Wie sich insbesondere aus der Darstellung nach Figur 1 ergibt, steht die Platte 22 über die Tragrahmenkonstruktion 14 zumindest abschnittsweise über und bildet insbesondere im Bereich einer jeden Tragrolle 3 Überstände 24 aus. Diese Überstände 24 dienen als Rammschutz.

Wie sich insbesondere aus der Darstellung nach Figur 1 ergibt, stehen der frontseitige Zugang zum Schrankunterteil 2 und die Blickrichtung auf den Monitor 8 unter einem 90° Winkel. Dies erleichtert einem Verwender die Arbeitsweise, denn es ist für einen Zugriff auf das Schrankunterteil 2 nicht erforderlich, die Arbeitsstellung vor dem Schrankoberteil 4 zu verlassen. In der typischen Arbeitsposition vor dem Monitor 8 können die Schubladen bestimmungsgemäß geöffnet und geschlossen werden, und zwar mit Bezug auf die Blickrichtung auf den Monitor 8 für ein Öffnen nach rechts bzw. für ein Schließen nach links.

Um insbesondere ein Bedienen der Rechneranlage im Sitzen zu erleichtern, ist die Platte 22 zwischen den beiden Tragrollen 3 tailliert ausgebildet. In gleicher Weise ist auch die der Platte 22 gegenüberliegend und die Tragrahmenkonstruktion 14 oberseitig verschließende Abdeckplatte ebenfalls tailliert ausgebildet.

Der in Figur 1 dargestellte Stationswagen stellt eine sogenannte Mischlösung dar. Einerseits ist er dank der mitgeführten Rechnereinheit dafür ausgelegt, im rein digitalen Betrieb verwendet zu werden. Andererseits bietet das Schrankunterteil 2 hinreichend Volumenraum, um auch noch Papierdokumente, beispielsweise Patientenakten mitführen zu können. Der erfindungsgemäße Stationswagen 1 eignet sich deshalb sowohl für den analogen als auch für den digitalen Betrieb.

### Bezugszeichen

- 1: Stationswagen
- 2: Schrankunterteil
- 3: Tragrolle
- 4: Schrankoberteil
- 5: Höhenrichtung
- 6: Säule
- 7: Säule
- 8: Monitor
- 9: Abdeckplatte
- 10: Staufach
- 11: Staufach
- 12: Schublade
- 13: Schublade
- 14: Tragrahmenkonstruktion
- 15: Stützstreben
- 16: Stützstreben
- 17: Stützstreben
- 18: Stützstreben
- 19: Profilelement
- 20: Rollenbock
- 21: Zapfen
- 22: Platte
- 23: Bohrungen
- 24: Überstände

## Patentansprüche

1. Stationswagen, insbesondere Visitenwagen für Krankenhäuser, Pflegeheime und sonstige Einrichtungen des Gesundheitswesens, mit einem mittels Tragrollen (3) fahrbar ausgebildeten Schrankunterteil (2), das der Aufnahme von Utensilien dient, und einem Schrankoberteil (3), das in Höhenrichtung (5) relativ verfahrbar zum Schrankunterteil (2) ausgebildet ist, **dadurch gekennzeichnet, dass** das Schrankunterteil (2) eine Tragrahmenkonstruktion (14) mit vertikal verlaufenden Stützstreben (15, 16, 17, 18) aufweist, wobei an jeder Stützstrebe (15, 16, 17, 18) jeweils ein Profilelement (19) angeordnet ist, das einen Volumenraum bereitstellt, in den ein Zapfen (21) einer Tragrolle (3) eingesteckt ist, wobei eine die Tragrahmenkonstruktion (14) tragrollenseitig abdeckende Platte (22) vorgesehen ist, die zwischen den tragrollenseitigen Enden der Profilelemente (19) und einem Rollenbock (20) der jeweiligen an dem Profilelement (19) angeordneten Tragrolle (3) angeordnet ist.

2. Stationswagen nach Anspruch 1, **dadurch gekennzeichnet, dass** die Platte (22) die Tragrahmenkonstruktion (14) unter Ausbildung von Überständen (24) seitlich zumindest abschnittsweise überragt.

3. Stationswagen nach Anspruch 2, **dadurch gekennzeichnet, dass** die Platte (22) im Bereich einer jeden Tragrolle (3) jeweils einen Überstand (24) bereitstellt.

4. Stationswagen nach einem der vorhergehenden Ansprüche, **dadurch gekennzeichnet, dass** die Tragrahmenkonstruktion plattenseitig einen zur Platte (22) beabstandet angeordneten Innenboden trägt.

5. Stationswagen nach Anspruch 4, **dadurch gekennzeichnet, dass** der zwischen Platte (22) und Innenboden befindliche Hohlraum der Kabelverlegung dient.

## Claims

1. A trolley, in particular ward trolley for hospitals, nursing homes and other health care establishments, comprising a bottom part of a cabinet (2) that is movable by means of support rollers (3), which bottom part serves to receive utensils, and an upper part of the cabinet (3) which can be displaced in the direction of height (5) with respect to the bottom part of the cabinet (2), **characterized in that** the bottom part of the cabinet (2) comprises a support frame construction (14) having vertically extending supporting struts (15, 16, 17, 18), wherein respectively one profile element (19) is arranged on each supporting strut (15, 16, 17, 18), which profile element (19) provides a volume space, into which a pin (21) of a support roller (3) is inserted, wherein a plate (22) is provided which covers the supporting frame construction (14) on the side of the support rollers, which plate (22) is arranged between the ends of the profile elements (19) on the side of the support rollers and a roller block (20) of the respective support roller (3) arranged on the profile element.

2. A trolley according to claim 1, **characterized in that** the plate (22) laterally projects over at least some sections of the supporting frame construction (14) while forming protrusions (24).

3. A trolley according to claim 2, **characterized in that** the plate (22) provides respectively one protrusion (24) in the area of each support roller (3).

4. A trolley according to one of the preceding claims, **characterized in that** on the side of the plate the supporting frame construction carries an inner bottom spaced from the plate (22).

5. A trolley according to claim 4, **characterized in that** the hollow space formed between the plate (22) and the inner bottom serves for laying cables.

## Revendications

1. Chariot médical, notamment chariot de visites, destiné à des hôpitaux, des établissements de soins ou à d'autres établissements de santé, comprenant une partie inférieure d'armoire (2), qui est conçue mobile grâce à des rouleaux porteurs (3) et qui sert à recevoir des ustensiles, et une partie supérieure d'armoire (3), qui est déplaçable dans la direction de la hauteur par rapport à la partie inférieure d'armoire (2), **caractérisé en ce que** la partie inférieure d'armoire (2) comprend une construction de cadre de support (14) comprenant des barres d'appui (15, 16, 17, 18), qui s'étendent verticalement, dans lequel respectivement un élément profilé (19) est disposé sur chaque barre d'appui (15, 16, 17, 18), les éléments profilés (19) fournissant chacun un espace de volume, dans lequel est inséré un tenon (21) d'un rouleau porteur (3), une plaque (22) étant prévue, qui couvre la construction de cadre de support (14) sur le côté des rouleaux porteurs, laquelle plaque (22) est disposée entre les extrémités des éléments profilés (19) sur le côté des rouleaux porteurs et un support à rouleaux (20) du rouleau porteur (3) respectif disposé sur l'élément profilé (19).

2. Chariot médical selon la revendication 1, **caractérisé en ce que** la plaque (22) surplombe au moins plusieurs sections de la construction de cadre de support (14) latéralement en formant des saillies (24).

3. Chariot médical selon la revendication 2, **caractérisé en ce que** la plaque (22) fournit respectivement une saillie (24) dans la zone de chaque rouleau porteur (3).

4. Chariot médical selon l'une des revendications précédentes, **caractérisé en ce que** la construction de cadre de support porte, sur le côté de la plaque, un sol intérieur espacé de la plaque (22).

5. Chariot médical selon la revendication 4, **caractérisé en ce que** la cavité, qui se trouve entre la plaque (22) et le sol intérieur, sert à poser des câbles.
